# EUROPEAN PATENT SPECIFICATION

(11) **EP 3 600 103 B1**
(45) Date of publication and mention of the grant of the patent: **01.01.2025**
(21) Application number: 18772459.6
(22) Date of filing: 15.03.2018
(51) Int. Cl.: A61B 18/14, A61N 1/05

(54) **THROMBECTOMY USING BOTH ELECTROSTATIC AND SUCTION FORCES**
THROMBEKTOMIE UNTER VERWENDUNG VON ELEKTROSTATISCHEN SOWIE SAUGKRÄFTEN
THROMBECTOMIE UTILISANT À LA FOIS DES FORCES ÉLECTROSTATIQUES ET D'ASPIRATION

(30) Priority: 22.03.2017 US 201762474628 P
(43) Date of publication of application: 05.02.2020
(73) Proprietor: Magneto Thrombectomy Solutions Ltd., 6037604 Or Yehuda (IL)
(72) Inventor: TAFF, Yuval, 6299622 Tel Aviv (IL); STERN, Gal, 6937247 Tel Aviv (IL); ORION, Itzhak, 8470110 Beer Sheva (IL)
(74) Representative: Beck Greener LLP
(86) International application number: PCT/IB2018/051731
(87) International publication number: WO 2018/172891

(56) References cited:
- EP-A1- 3 071 280
- WO-A1-2014/025397
- WO-A1-2014/025397
- DE-A1- 102010 014 778
- US-A1- 2007 027 448
- US-A1- 2011 301 594
- US-A1- 2016 066 989

## Description

Embodiments of the present invention relate to the treatment of thrombi in the body of a subj ect.

US2011/0301594 describes a flexible catheter device capable of being introduced into body passages, and withdrawing fluids therefrom or introducing fluids thereinto. The device includes electrodes configured to apply electrical voltage signals in the body passage for carrying out thrombus dissolution and/or thrombectomy, wherein one of said electrodes is designed to contact the thrombus material and remove it or dissolve it, and wherein the electrical voltage signals include a unipolar pulsatile voltage signal. US2001/0001314 describes electrosurgical apparatus and methods for maintaining patency in body passages subject to occlusion by invasive tissue growth. The apparatus includes an electrode support disposed at a shaft distal end having at least one active electrode arranged thereon, and at least one return electrode proximal to the at least one active electrode. In one embodiment, a plurality of active electrodes each comprising a curved wire loop portion are sealed within a distal portion of the electrode support. US2004/0073243 describes devices and methods for removing an obstruction from a blood vessel. The devices are deployed in a collapsed condition and are then expanded within the body. The devices are then manipulated to engage and remove the obstruction. WO201402537 discloses a low-profile electrode assembly that has an inner electrode, an insulating layer disposed over the inner electrode such that an opening in the insulating layer is aligned with the inner electrode, and an outer electrode sheath disposed over the insulating layer such that an opening in the outer electrode sheath is coaxially aligned with the opening in the insulating layer. EP3071280 discloses catheters that have therapeutic agent and therapeutic energy delivery capability. US2016066989 discloses an electrosurgical device comprising an electrically conductive elongate member for traversing body vasculature defining a hollow lumen with one or more apertures at or near its distal end, wherein electrical energy can flow through the wall of the elongate member; and an energy delivery device in electrical communication with the elongate member located at or about the distal end of the elongate member. US2007027448 discloses bipolar and multipolar, virtual-electrode catheters having at least one internal electrode and at least one surface electrode, and methods of using these catheters for treatment of cardiac arrhythmias via, for example, radiofrequency (RF) ablation.

There is provided, in accordance with an aspect of the present invention, an apparatus as set out in the appending independent claim. Features of different embodiments are set out in the appending dependent claims Methods disclosed herein do not form part of the claimed invention.

There is also disclosed herein examples of an apparatus for removal of a thrombus from a body of a subject. The apparatus includes an electrically-insulating tube, which includes a distal end having a circumferential wall that is shaped to define one or more perforations, configured for insertion into the body of the subject. The apparatus further includes an outer electrode, disposed over the distal end of the electrically-insulating tube, and configured to lie at least partly within the thrombus while the electrically-insulating tube is inside the body of the subject. The apparatus further includes an inner electrode, configured to lie, within the tube, opposite the perforations, while the outer electrode lies at least partly within the thrombus. The outer electrode is configured to attract the thrombus while the outer electrode lies at least partly within the thrombus and the inner electrode lies opposite the perforations, when a positive voltage is applied between the outer electrode and the inner electrode such that electric current flows through the perforations.

In some examples, the distal end of the electrically-insulating tube is shaped to define a distal opening.

In some examples, the distal end of the electrically-insulating tube is closed.

In some examples, the distal end of the electrically-insulating tube tapers distally to the perforations.

In some examples, the apparatus further includes a pump, configured to facilitate the removal of the thrombus by applying suction to the thrombus, via the perforations, while the voltage is applied.

The outer electrode includes a coil that wraps around the distal end of the electrically-insulating tube.

The coil wraps around the distal end of the electrically-insulating tube between the perforations.

In some examples, the perforations are first perforations, and the outer electrode includes an electrically-conducting tube shaped to define one or more second perforations that are aligned with the first perforations.

In some examples, the perforations are arranged in one or more rows.

In some examples, the rows of perforations are distributed circumferentially along the circumferential wall.

In some examples, for each of the rows, at least one of the perforations in the row is larger than other perforations in the row.

In some examples, at least a proximalmost one of the perforations in the row is larger than the other perforations in the row.

In some examples, the inner electrode includes a wire.

In some examples, the inner electrode includes an electrically-conducting coil.

In some examples, the inner electrode includes an electrically-conducting mesh.

In some examples, the inner electrode includes an electrically-conducting tube.

In some examples, the perforations are first perforations, and the electrically-conducting tube is shaped to define one or more second perforations that are aligned with the first perforations.

There is also disclosed herein other examples of an apparatus for removal of a thrombus from a body of a subject. The apparatus includes an electrically-insulating tube, which includes a distal end shaped to define a distal opening, configured for insertion into the body of the subject. The apparatus further includes an outer electrode, disposed over the distal end of the electrically-insulating tube, and configured to lie at least partly within the thrombus while the electrically-insulating tube is inside the body of the subject. The apparatus further includes an inner electrode, configured to lie at least partly within the distal end of the electrically-insulating tube while the outer electrode lies at least partly within the thrombus. The outer electrode is configured to attract the thrombus while the outer electrode lies at least partly within the thrombus and the inner electrode lies at least partly within the distal end of the electrically-insulating tube, when a positive voltage is applied between the outer electrode and the inner electrode.

In some examples, the apparatus further includes a pump, configured to facilitate the removal of the thrombus by aspirating any detached portions of the thrombus through the distal opening.

In some examples, the outer electrode includes a coil that wraps around the distal end of the electrically-insulating tube.

In some examples, the outer electrode includes an electrically-conducting tube.

In some examples, the inner electrode includes a wire.

In some examples, the inner electrode includes an electrically-conducting tube.

In some examples, the inner electrode includes an electrically-conducting coil.

In some examples, the inner electrode includes an electrically-conducting mesh.

There is further disclosed herein examples of a method for removing a thrombus from a body of a subject. The method includes inserting an electrically-insulating tube, which includes a distal end having a circumferential wall that is shaped to define one or more perforations, into the body of the subject. The method further includes, subsequently to inserting the electrically-insulating tube, navigating the electrically-insulating tube through the body of the subject, until an outer electrode, which is disposed over the distal end of the electrically-insulating tube, lies at least partly within the thrombus. The method further includes, while the outer electrode lies at least partly within the thrombus, applying a positive voltage between the outer electrode and an inner electrode that is disposed, within the tube, opposite the perforations, such that electric current flows through the perforations, causing the thrombus to adhere to the outer electrode. The method further includes, while the thrombus adheres to the outer electrode, removing the thrombus from the body of the subject, by withdrawing the electrically-insulating tube.

In some examples, applying the positive voltage includes continuing to apply the positive voltage while withdrawing the electrically-insulating tube.

In some examples, the method further includes, while the outer electrode lies at least partly within the thrombus, applying suction to the thrombus via the perforations.

In some examples, applying the suction includes continuing to apply the suction while withdrawing the electrically-insulating tube.

In some examples, the distal end is shaped to define a distal opening, and the method further includes, by applying the suction, aspirating any detached portions of the thrombus through the distal opening.

In some examples, at least some of the perforations are arranged in a row, a largest perforation in the row being larger than other perforations in the row, navigating the electrically-insulating tube through the body of the subject includes navigating the electrically-insulating tube through the body of the subject until the largest perforation is aligned with a proximal end of the thrombus, and applying the positive voltage includes applying the positive voltage while the largest perforation is aligned with the proximal end of the thrombus.

In some examples, the inner electrode includes a wire, the method further includes, prior to navigating the electrically-insulating tube through the body of the subject, passing the wire through the thrombus, and navigating the electrically-insulating tube through the body of the subject includes navigating the electrically-insulating tube over the wire.

In some examples, the method further includes, prior to applying the voltage, withdrawing the wire into the electrically-insulating tube.

In some examples, applying the positive voltage includes applying the positive voltage while a distal tip of the inner electrode is aligned with a distalmost one of the perforations.

There is further disclosed herein some other examples of a method for removing a thrombus from a body of a subject. The method includes inserting an electrically-insulating tube, which includes a distal end shaped to define a distal opening, into the body of the subject. The method further includes, subsequently to inserting the electrically-insulating tube, navigating the electrically-insulating tube through the body of the subject, until an outer electrode, which is disposed over the distal end of the electrically-insulating tube, lies at least partly within the thrombus. The method further includes, while the outer electrode lies at least partly within the thrombus, applying a positive voltage between the outer electrode and an inner electrode that is disposed at least partly within the distal end of the electrically-insulating tube, such that electric current flows between the outer electrode and the inner electrode, causing the thrombus to adhere to the outer electrode. The method further includes, while the thrombus adheres to the outer electrode, removing the thrombus from the body of the subject by withdrawing the electrically-insulating tube while aspirating any detached portions of the thrombus through the distal opening.

In some examples, applying the positive voltage includes continuing to apply the positive voltage while withdrawing the electrically-insulating tube.

In some examples, the inner electrode includes a wire, the method further includes, prior to navigating the electrically-insulating tube through the body of the subject, passing the wire through the thrombus, and navigating the electrically-insulating tube through the body of the subject includes navigating the electrically-insulating tube over the wire.

In some examples, the method further includes, prior to applying the voltage, withdrawing the wire into the electrically-insulating tube.

The present invention will be more fully understood from the following detailed description of embodiments thereof, taken together with the drawings, in which:
Figs. 1A-B are schematic illustrations of an apparatus for removing a thrombus from a body of a subject;
Fig. 2 is a longitudinal cross-section through part of an electrically-insulating tube;
Figs. 3A-B are schematic illustrations of the distal end of an electrically-insulating tube;
Fig. 4 is a schematic illustration of an apparatus for removing a thrombus from a body of a subject;
Fig. 5 is a schematic illustration showing the delivery of an apparatus to a thrombus, which is located within a blood vessel of a subject; and
Fig. 6 is a flow diagram for a method for removing a thrombus from a body of a subject.

### OVERVIEW

Embodiments of the present invention include apparatuses and methods for a thrombectomy, i.e., for the removal of a thrombus from the body of a subject. The thrombus may include any material that at least partly occludes a passageway, such as a blood vessel, within the subject's body. For example, the thrombus may include coagulated blood, fat, cholesterol, plaque, or any foreign material originating from outside the body.

In general, the thrombectomy techniques described herein involve applying a voltage between two electrodes that are radially separated from one another. For example, one thrombectomy device described herein comprises an outer electrode disposed over the distal end of an electrically-insulating tube, which in turn contains an inner electrode. The wall of the tube is shaped to define one or more perforations, which are disposed beneath the outer electrode. The device is advanced through the blood vessel, until the outer electrode is lodged (or "embedded") within the thrombus. Subsequently, a positive voltage is applied between the outer and inner electrodes, such that electric current flows between the electrodes via the perforations, causing the negatively-charged thrombus to adhere to the positively-charged outer electrode. While the thrombus adheres to the outer electrode, the device, along with the thrombus, is withdrawn from the subject.

Advantageously, due to the radial separation between the two electrodes, along with the perforations in the tube that allow for the flow of electric current therethrough, relatively little current flows through the blood vessel and the nearby tissue. In contrast, if the two electrodes were longitudinally (or "axially") separated from one another during the application of the voltage, or if the wall of the tube were not perforated, more current might need to flow through the blood vessel and the nearby tissue. Furthermore, since the outer electrode is disposed over the tube, the outer electrode may contact the thrombus over a relatively large surface area, facilitating the application of a relatively large attractive electrostatic force to the thrombus. In contrast, if the outer electrode were carried to the thrombus within the tube and then advanced from the tube into the thrombus, the outer electrode might need to have a smaller profile, such as to fit within the tube.

Another advantage of the perforations is that a suction force may be applied, via the perforations, to the thrombus, thus causing the thrombus to be more strongly attached to the thrombectomy device. In addition to applying suction through the side perforations, suction may be applied through the distal opening of the tube, such as to aspirate any bubbles or debris that collect while the voltage is applied and/or while the device is withdrawn. Advantageously, since the outer electrode is disposed over the tube, the outer electrode does not block the distal opening of the tube, such that bubbles and debris may be more effectively aspirated through the distal opening.

In some cases, the location of the proximal end of the thrombus is known, but the exact length of the thrombus is unknown. In such cases, if the perforations were uniformly sized, the suction and electrostatic forces might be extensively applied over portions of the tube that are not situated within the thrombus. Hence, in some embodiments, the perforations are not uniformly sized. Rather, one of the perforations is larger than the other perforations, and this largest perforation is aligned with the proximal end of the thrombus. For example, the most proximal perforation may be larger than the more distal perforations, and this perforation may be aligned with the proximal end of the thrombus.

In other embodiments, the electrically-insulating tube is not perforated. In such embodiments, the applied suction force is concentrated at the distal opening of the tube, such as to provide better aspiration of any bubbles or debris generated during the procedure, relative to if the tube were perforated.

In some embodiments, the inner electrode comprises a wire that runs through the length of the tube. In such embodiments, the inner electrode may additionally function as a guidewire, to facilitate advancement of the tube through the vasculature of the subject. In other embodiments, the inner electrode comprises an inner, electrically-conducting tube having perforations that are aligned with those of the outer, electrically-insulating tube.

### APPARATUS DESCRIPTION

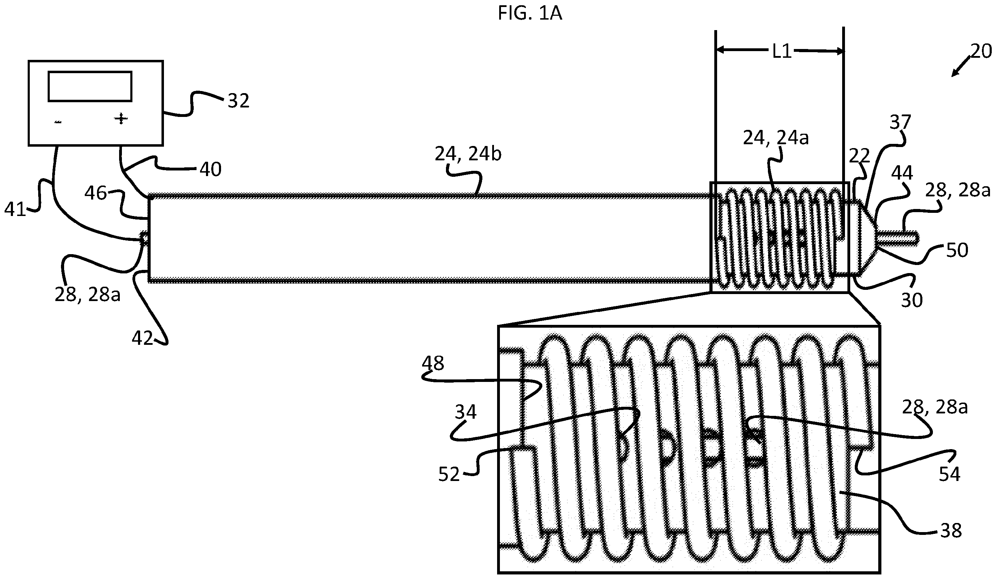

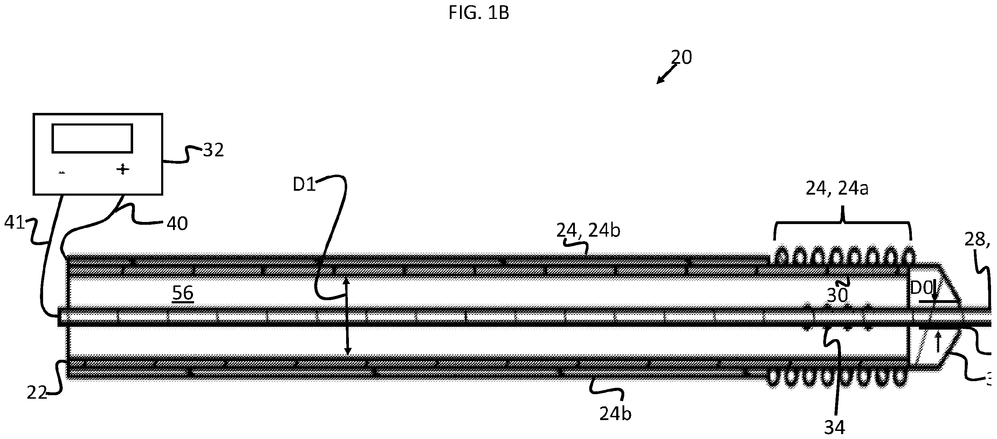

Reference is initially made to Figs. 1A-B, which are schematic illustrations of an apparatus 20 for removing a thrombus from a body of a subject, in accordance with some embodiments of the present invention. (Fig. 1B shows a longitudinal cross-section through apparatus 20.)

Apparatus 20 comprises an electrically-insulating tube 22, which is configured for insertion into the body of a subject. Tube 22 may be made of any suitable biocompatible insulating material, such as silicone, polyurethane, polyethylene, or polytetrafluoroethylene. Tube 22 comprises a distal end 30 having a circumferential wall 38 that is shaped to define one or more perforations 34. (Hence, tube 22 may be referred to as a "perforated tube.") Tube 22 is shaped to define an inner lumen 56, which is surrounded by the circumferential wall of the tube.

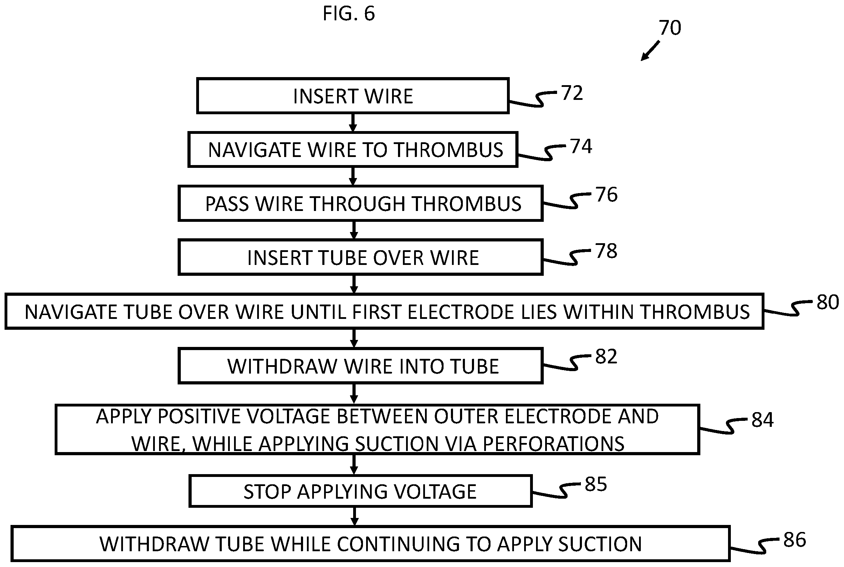

Apparatus 20 further comprises an outer electrode 24, which is disposed over at least part of distal end 30, and an inner electrode 28, which is configured to lie within inner lumen 56. A first wire 40 connects outer electrode 24 to the positive terminal of a voltage source 32, which is disposed externally to the subject, while a second wire 41 connects inner electrode 28 to the negative terminal of the voltage source. As further described below with reference to Fig. 6, while outer electrode lies at least partly within the thrombus and inner electrode 28 lies opposite perforations 34 (e.g., with the distal tip of the inner electrode being aligned with the distalmost perforation), voltage source 32 applies a positive voltage between outer electrode 24 and inner electrode 28. The positive voltage causes the outer electrode to attract the negatively-charged thrombus, such that the thrombus adheres to the outer electrode.

Typically, each of the electrodes is constructed of a highly conductive biocompatible metal. To increase the effectivity of the applied positive voltage, the outer electrode may have a higher electronegativity than that of the inner electrode. Thus, for example, the outer electrode may be made of gold, platinum, or any alloy thereof (such as an alloy of platinum and iridium), while the inner electrode may be made of stainless steel, Nitinol, or titanium.

In some embodiments, at least one of the electrodes is at least partly radiopaque, and/or comprises one or more radiopaque markers, to facilitate navigation under fluoroscopy. Similarly, distal end 30 may comprise one or more radiopaque markers, to facilitate navigation of tube 22 under fluoroscopy.

In some embodiments, as shown in Fig. 1A, outer electrode 24 comprises two portions: a distal portion 24a, which wraps around distal end 30 of the tube, and a proximal portion 24b. Proximal portion 24b is distally connected to distal portion 24a and proximally connected to first wire 40, such that distal portion 24a is connected to voltage source 32 via proximal portion 24b. Typically, the length L1 of distal portion 24a is between 5 and 150 mm, such as between 10 and 50 mm. The proximal end 52 of distal portion 24a is typically positioned proximally to the proximalmost perforation 34, while the distal end 54 of distal portion 24a is typically positioned distally to the distalmost perforation 34.

In some embodiments, the distal portion of the outer electrode comprises a coil, of a constant or variable pitch, that coils around distal end 30. Alternatively, the distal portion of outer electrode 24 may comprise an electrically-conductive braid or mesh that is wrapped around distal end 30. (In some cases, the distal portion of the outer electrode may partially cover perforations 34, as shown in Fig. 1A.) The proximal portion of the outer electrode may comprise an electrically-conducting tube that passes over tube 22, and/or an electrically-conducting braid, mesh, or coil that wraps around tube 22. The proximal portion of the outer electrode may be made of any suitable material, such as stainless steel, Nitinol, or titanium, and may be coated for improved lubricity.

In other embodiments, outer electrode 24 comprises a single electrically-conductive tube, mesh, braid, or coil that is proximally connected to first wire 40, and extends from the proximal end of tube 22 over at least some of distal end 30. For embodiments in which the outer electrode comprises a tube, the tube may be shaped to define one or more perforations that are aligned with perforations 34.

Typically, the outer electrode is affixed to tube 22, such that the outer electrode does not slide along the tube. In some embodiments, however, the outer electrode may slide along tube 22.

In some embodiments, inner electrode 28 comprises a wire 28a. In such embodiments, wire 28a may facilitate guiding tube 22 to the thrombus, in addition to facilitating the aforementioned application of voltage. For example, wire 28a may be inserted into the subject, and then navigated, under fluoroscopic imaging or any other suitable imaging technique, to the thrombus. Next, wire 28a may be passed through the thrombus. Subsequently, tube 22 (and outer electrode 24) may be passed over the wire, until the outer electrode is disposed within the thrombus.

Alternatively, a separate guidewire may be used to guide tube 22 to the thrombus. After the outer electrode is lodged within the thrombus, the guidewire may be withdrawn, and wire 28a may then be passed through tube 22, to the distal end of the tube.

During the application of voltage between wire 28a and the outer electrode, the distal portion of the wire is positioned within the distal end of the tube, opposite (or "behind") the perforations, such that the electric current that flows between the electrodes may pass through the perforations. For example, the distal tip of the wire may be aligned with the distalmost perforation. In some embodiments, most of wire 28a is coated with an insulating material, with only the distal portion of the wire being uncoated.

The diameter D1 of inner lumen 56 of tube 22 is typically between 0.25 and 5 mm, such as between 0.4 and 2 mm. Such a diameter is generally large enough to allow the passage of electrode 28, along with any aspirated debris, through tube 22, yet is small enough to facilitate navigation of the tube through the vasculature of the subject.

In some embodiments, distal end 30 is shaped to define a distal opening 50. In some such embodiments, distal opening 50 is sufficiently wide such that, while the voltage is applied between the electrodes, any detached portions of the thrombus may be aspirated through distal opening 50 by the suitable application of suction through tube 22. (In other words, the distal opening may function as an aspiration port.) For example, the diameter D0 of distal opening 50 may be approximately equal to diameter D1 of the tube lumen. In other such embodiments, D0 is significantly less than D1, such that the suction force applied through tube 22 is concentrated at perforations 34, thus facilitating better capture of the thrombus. For example, D0 may be approximately the same as that of wire 28a (or that of a typical guidewire), e.g., between 0.2 and 1 mm, with D1 being at least twice D0.

In other embodiments, distal end 30 is closed, i.e., distal end 30 is not shaped to define a distal opening, such that the applied suction force is fully concentrated at perforations 34. In such embodiments, apparatus 20 is advanced without the use of a guidewire. For example, apparatus 20 may be passed through a catheter. Alternatively, tube 22 may be shaped to define another lumen in addition to lumen 56, and rapid exchange techniques may be used to position the apparatus.

In some embodiments, distal end 30 tapers distally to the perforations, such that distal end 30 comprises a pointy distal tip configured to penetrate the thrombus. For example, the inner diameter of distal end 30 may decrease from D1 to D0, where D0 is less than 50% of D1, over a length of 1-3 mm. As shown in Fig. 1A, distal end 30 may taper distally by virtue of comprising a tapered end cap 37, which is fastened to the main body of tube 22.

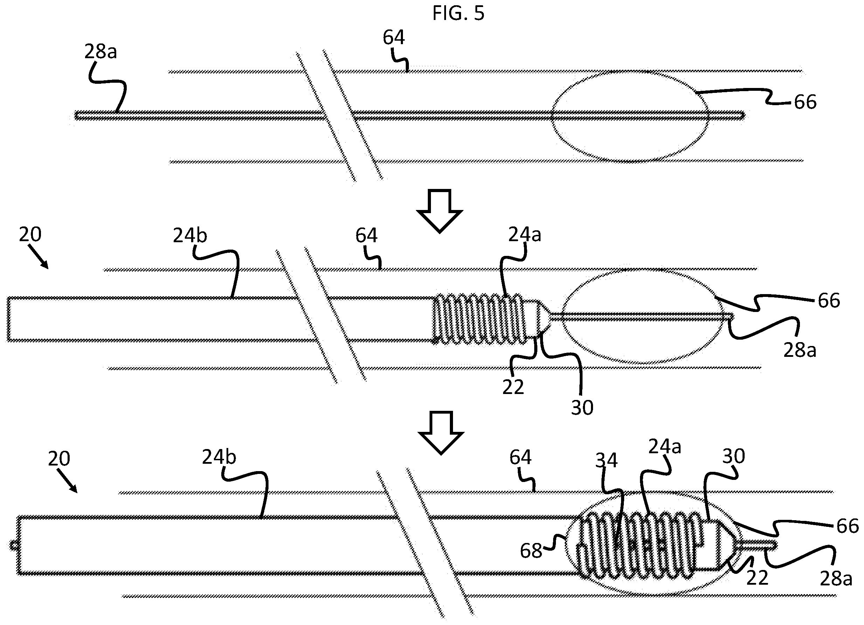

It is noted that even in embodiments in which distal opening 50 is significantly narrowed or non-existent, the suction force that is applied may perform an aspirating function, in addition to facilitating the capture of the thrombus. For example, as further described below with reference to Fig. 5, the applied suction force may aspirate bubbles that form near the inner electrode while the voltage is applied, provided that these bubbles form within the tube.

It is noted that the various tubes, wires and other longitudinal elements described herein are typically flexible, so as to facilitate passage of these elements through the vasculature of the subject. Each of these elements may have any suitable length, so as to allow the removal of a thrombus from any blood vessel belonging to the subject. For example, the distance between the proximal tip 42 of tube 22 and the distal tip 44 of tube 22 (i.e., the distal tip of distal end 30) may be between 20 and 200 cm. (In view of the above, it is noted that Fig. 1A is not drawn to scale, in that the distal portion of apparatus 20 is drawn disproportionately large.)

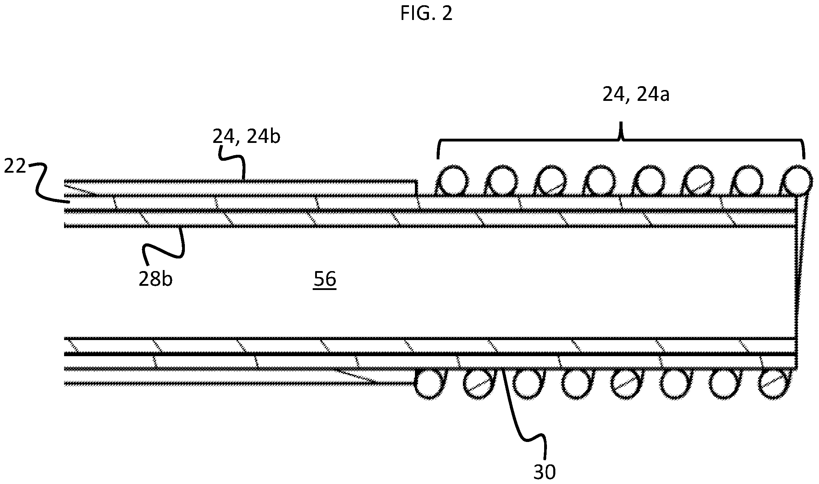

Reference is now made to Fig. 2, which is a longitudinal cross-section through part of tube 22, in accordance with some embodiments of the present invention.

In some embodiments, as shown in Fig. 2, the inner electrode comprises an electrically-conducting tube (or "shaft") 28b, instead of wire 28a. Typically, in such embodiments, a separate guidewire guides the delivery of apparatus 20 to the thrombus. (As in Figs. 1A-B, tube 22 may comprise end cap 37.)

Typically, electrically-conducting tube 28b is affixed to the inner wall of the electrically-insulating tube. In such embodiments, tube 28b is typically shaped to define one or more perforations that are aligned with perforations 34 in tube 22. In other embodiments, electrically-conducting tube 28b is slidably disposed within electrically-insulating tube 22. As described above for wire 28a, most of tube 28b may be coated with an insulating material, with only the distal portion of tube 28b - which may be shaped to define the aforementioned perforations - being uncoated. Typically, the outer diameter of electrically-conducting tube 28b is approximately equal to inner diameter D1 of the tube, such that tube 28b adheres to, or slides along, the inner wall of tube 22.

In yet other embodiments, the inner electrode may comprise an electrically-conducting coil, braid, or mesh, which may be affixed to the inner wall of tube 22.

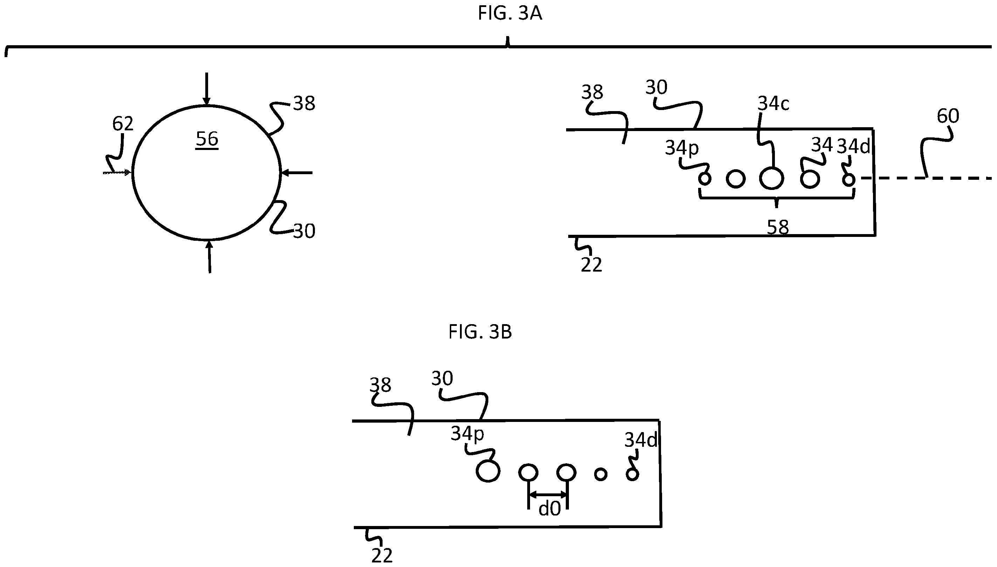

Reference is now made to Figs. 3A-B, which are schematic illustrations of distal end 30 of electrically-insulating tube 22, in accordance with some embodiments of the present invention. (Fig. 3A shows both a frontal view of distal end 30, at the left of the figure, and a side view of distal end 30, at the right of the figure.)

Typically, perforations 34 are arranged in one or more rows 58, each of which may have any suitable orientation. For example, at least one row 58 may be oriented parallel to the longitudinal axis 60 of distal end 30, in a proximal-distal direction. Distal end 30 may be shaped to define any suitable number of rows, and each of the rows may include any suitable number of perforations, such as between one and ten perforations.

Typically, rows 58 are distributed circumferentially along circumferential wall 38. For example, distal end 30 may be shaped to define four rows of circumferentially-distributed perforations, such that a 90-degree angle separates each pair of successive rows. Such an example embodiment is illustrated in the frontal view of Fig. 2A, whereby a plurality of arrows 62 indicate the respective positions of four rows 58. (It is noted that the rows are not necessarily at the same longitudinal position, i.e., one of the rows may be situated more distally than another one of the rows.)

In some embodiments, for each of the rows, at least one of the perforations in the row is larger than the other perforations in the row. For example, as shown in Fig. 3A, at least one of the perforations between the proximalmost perforation 34p and the distalmost perforation 34d, such as a central perforation 34c, may be larger than each of the other perforations in the row. Furthermore, the size of the perforations may decrease from the middle of the row, such that proximalmost perforation 34p and distalmost perforation 34d are smaller than each of the other perforations in the row. Alternatively, as shown in Fig. 3B, proximalmost perforation 34p may be larger than the other perforations in the row. In addition, the size of the perforations may decrease from the proximal end of the row, such that distalmost perforation 34d is the smallest perforation in the row.

Typically, the size of each perforation is between 7.5×10⁻⁵ and 10 mm². In some embodiments, for example, each perforation is circular, having a diameter of between 0.01 and 3.5 mm. For example, the largest perforation in each row may have a diameter of between 0.1 and 3.5 mm, with the smallest perforation have a diameter of between 0.01 and 0.3 mm. Alternatively or additionally, the combined size of the perforations in each row may be between 0.05 and 10 mm², and/or the combined size of all of the perforations, over all of the rows, may be between 0.2 and 20 mm². Alternatively or additionally, to prevent any pressure drops when suction is applied, this latter combined size may be larger than the circular cross-sectional area of lumen 56.

Typically, within any given row, each pair of successive perforations is separated by a distance d0 that is between 0.1 and 4 mm. In some embodiments, distance d0 varies across the row. For example, as shown in Fig. 3A, the distalmost perforation may be spaced from its proximally-neighboring perforation by a greater distance from that which separates the proximalmost perforation from its distally-neighboring perforation.

As further described below with reference to Fig. 6, while the outer electrode lies at least partly within the thrombus, suction is applied to the thrombus via the perforations. In general, the sizes of, and spacings between, the perforations may be matched to the location, size, and/or shape of the thrombus, to optimize the distribution of the electrostatic and suction forces applied to the thrombus. For example, as described above in the Overview, the largest perforations may be aligned with the proximal end of the thrombus.

Any suitable suction-applying device, such as a pump or syringe, may be used to apply the aforementioned suction force. Advantageously, provided that distal end 30 is open, the application of suction through tube 22 may further help aspirate bubbles and/or debris through distal opening 50.

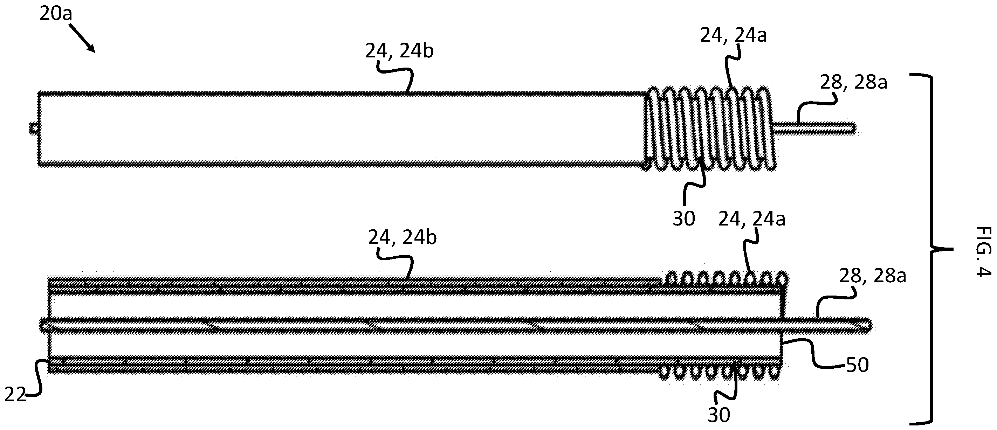

Reference is now made to Fig. 4, which is a schematic illustration of an apparatus 20a for removing a thrombus from a body of a subject, not according to the present invention. Fig. 3 includes both a side view of, and longitudinal cross-section through, apparatus 20a.

In several respects, the features of apparatus 20a are similar to those of apparatus 20. For example, in apparatus 20a, outer electrode 24 and inner electrode 28 are spaced radially from one another, with electrically-insulating tube 22 separating between the two electrodes. Similarly, each of the electrodes may have any of the forms described above, such that, for example, the outer electrode may comprise a coil, a mesh, a braid, and/or a tube, while the inner electrode may comprise a wire, a coil, a mesh, a braid, and/or a tube. Similarly, as in the case of apparatus 20, during the application of a voltage between the two electrodes, the inner electrode is positioned beneath the outer electrode, e.g., with the distal tip of the inner electrode being aligned with, or within several millimeters of, distal opening 50. Likewise, a pump or other suction-applying device may apply suction through tube 22 while the voltage is applied, and/or subsequently to the application of the voltage.

Apparatus 20a is different from apparatus 20, however, in that, in apparatus 20a, distal end 30 is not perforated. Due to the lack of perforations in distal end 30, electric current flows entirely through distal opening 50 while the voltage is applied between the electrodes. Advantageously, however, more suction force is concentrated at distal opening 50, such as to achieve better aspiration of any bubbles and debris that accumulate during the procedure.

Reference is now made to Fig. 5, which is a schematic illustration showing the delivery of apparatus 20 to a thrombus 66, which is located within a blood vessel 64 of a subject, in accordance with some embodiments of the present invention.

First, as shown in the top portion of Fig. 5, wire 28a is navigated, under fluoroscopy or any other suitable imaging modality, through the vasculature of the subject, until the wire reaches blood vessel 64. Subsequently, wire 28a is passed through thrombus 66. Next, as shown in the middle portion of Fig. 5, perforated tube 22 is inserted into the vasculature of the subject, and is then navigated, through the vasculature, to blood vessel 64. (Perforations 34 are not shown in this portion of the figure.) Subsequently, tube 22 is navigated through the vasculature, until, as shown in the bottom portion of Fig. 5, outer electrode 24, which is disposed over distal end 30, lies at least partly within the thrombus. (As described above with reference to Figs. 3A-B, in some embodiments, tube 22 may be navigated through the vasculature until the largest perforations are aligned with the proximal end 68 of the thrombus.)

Next, prior to applying voltage between the outer electrode and wire 28a, the wire may be withdrawn into tube 22, e.g., until the distal tip of the wire is aligned with the distalmost perforation(s). The withdrawal of the wire into the tube increases the proportion of the electric current that flows through the perforations while the voltage is applied. Furthermore, the withdrawal of the wire into the tube facilitates the aspiration of any bubbles that form near the wire, since most or all of these bubbles form within the tube.

The delivery of apparatus 20 to the thrombus, and the subsequent removal of the thrombus using apparatus 20, are hereby further described with reference to Fig. 6, which is a flow diagram for a method 70 for removing a thrombus from a body of a subject, in accordance with some embodiments of the present invention.

First, at a wire-inserting step 72, wire 28a is inserted into the subject. Subsequently, at a wire-navigating step 74, wire 28a is navigated to the thrombus. Next, at a thrombus-piercing step 76, wire 28a is passed through the thrombus, as described above with reference to the top portion of Fig. 5. Subsequently, at a tube-inserting step 78, tube 22 is inserted, over wire 28a, into the subject. Tube 22 is then navigated over the wire, at a tube-navigating step 80, until outer electrode 24 lies at least partly within the thrombus, as described above with reference to the middle and bottom portions of Fig. 5. Next, at a wire-withdrawing step 82, the wire is withdrawn into the tube, such that the distal end of the wire is disposed within the distal end of the tube, opposite the perforations.

Subsequently, at an applying step 84, voltage source 32 (Fig. 1) applies a positive voltage between the outer electrode and the wire, while a pump, or other suitable suction-applying device, applies suction to the thrombus via the perforations in the tube. The applied voltage causes electric current to flow through the perforations, such that the outer electrode attracts the thrombus. The applied suction supplements the applied voltage by pulling the thrombus radially inward, toward the tube, and may further aspirate any detached portions of the thrombus through the distal opening of the tube while the voltage is applied. In some embodiments, the application of suction begins before the application of voltage.

Typically, the voltage is applied for a duration that is sufficient for the thrombus to adhere to the outer electrode, without exposing the subject to any unnecessary risk. For example, this duration may be between one second and 10 minutes, such as between 5 seconds and 5 minutes, e.g., between 10 seconds and 2 minutes. Following this duration, the application of the voltage is stopped, at a voltage-stopping step 85. Finally, at a tube-withdrawing step 86, while the thrombus adheres to the outer electrode, the thrombus is removed from the body of the subject, by withdrawing the tube, over the wire, from the subject. (In some embodiments, the wire is withdrawn along with the tube, while in other embodiments, the wire is withdrawn only after the tube has been withdrawn.) While the tube is withdrawn, the application of suction continues, such as to help keep the thrombus attached to the outer electrode, and/or aspirate any detached portions of the thrombus through the distal end of the tube.

In alternate embodiments, the application of the voltage may continue during at least part of the time during which the tube is withdrawn.

As noted above, in some embodiments, the inner electrode does not comprise wire 28a, or wire 28a does not function as a guidewire. In such embodiments, a separate guidewire, instead of wire 28a, is inserted into the subject at wire-inserting step 72. Method 70 then proceeds as outlined above, until the completion of tube-navigating step 80. Following tube-navigating step 80, the guidewire is withdrawn from the body, and the inner electrode is then inserted into tube 22, until the distal end of the inner electrode is aligned with the distal perforations in the tube. Subsequently, applying step 84, along with the subsequent steps, are performed, as outlined above.

It is noted that the technique described above with reference to Fig. 6 may be used with apparatus 20a (Fig. 4), *mutatis mutandis.* For example, further to positioning the apparatus as described above, a suction force may be applied together with the positive voltage, and/or prior to or following the application of the voltage. Although this suction force does not draw the thrombus radially inward (due to the lack of perforations in the tube), this suction force may nonetheless aspirate any detached portions of the thrombus through the distal opening of the tube.

In general, the positive voltage signal that is applied between the electrodes may have any suitable form, such as any of the forms described in US2011/0301594. For example, the voltage signal may be a periodic signal that includes a sequence of pulses, each of these pulses, for example, being shaped as the positive half-wave of a sinusoidal signal, or having a trapezoidal shape. Alternatively, the voltage signal may be a direct current (DC) voltage signal. Typically, the voltage signal is unipolar. Although the amplitude of the voltage may have any suitable value, this amplitude is typically between 0.1 and 100 V, e.g., between 1 and 100 V, such as between 1 and 50 V, or between 4 and 40 V. Such an amplitude is large enough to be effective, yet small enough such as to avoid damaging the tissue near the thrombus.

For example, as described in US2011/0301594 with reference to Fig. 1D thereof, each trapezoidal pulse of the applied voltage signal may (i) linearly ramp up from ground level (0 volts) to an amplitude of around 40 volts, over a time period of around 5 milliseconds, (ii) remain constant over a time period of around 5 milliseconds, and then (iii) linearly ramp down to ground level over a time period of around 5 milliseconds. Before the beginning of the subsequent pulse, the voltage may remain at ground level for another time period of around 5 milliseconds. In general, the applied voltage signal, if pulsatile, may have any suitable frequency, such as between 0.1 Hz to 100 MHz, e.g., around 50 Hz, as in the example immediately above. Typically, the voltage is applied such that a current having an amplitude of between 0.1 and 4 mA (e.g., 1-3 mA) is passed between the two electrodes.

In some embodiments, voltage source 32 is current-regulated, e.g., to between 0.1 and 4 mA, such as 1-3 mA. In other embodiments, the voltage source is voltage-regulated, e.g., to between 0.1 and 100 V, or any of the smaller subranges outlined above.

In alternate embodiments, when using apparatus 20a, a negative voltage, which dissolves the thrombus, is applied between the outer and inner electrodes, by connecting the outer electrode to the negative terminal of voltage source 32, and connecting the inner electrode to the positive terminal. As the negative voltage is applied, the dissolved pieces of thrombus material are aspirated though the distal opening of tube 22.

## Claims

1. Apparatus (20) for removal of a thrombus (66) from a body of a subject, the apparatus comprising:
an electrically-insulating tube (22), which comprises a distal end (30) having a circumferential wall (38) that is shaped to define multiple perforations (34) arranged in one or more rows (58), configured for insertion into the body of the subject;
an outer electrode (24), disposed over the distal end of the electrically-insulating tube without entirely covering the perforations such that a proximal electrode-end (52) of the outer electrode is proximal to a proximalmost one of the perforations and a distal electrode-end (54) of the outer electrode is distal to a distalmost one of the perforations, and configured to lie at least partly within the thrombus while the electrically-insulating tube is inside the body of the subject; and
an inner electrode (28), configured to lie, within the tube, opposite the perforations, while the outer electrode lies at least partly within the thrombus;
the outer electrode being configured to attract the thrombus while the outer electrode lies at least partly within the thrombus and the inner electrode lies opposite the perforations, when a positive voltage is applied between the outer electrode and the inner electrode such that electric current flows through the perforations;
**characterized in that** the outer electrode comprises a coil that wraps around the distal end of the electrically-insulating tube; and
**in that** the coil wraps around the distal end of the electrically-insulating tube between the perforations.

2. The apparatus according to claim 1, wherein the distal end of the electrically-insulating tube is shaped to define a distal opening.

3. The apparatus according to claim 1, wherein the distal end of the electrically-insulating tube is closed.

4. The apparatus according to any preceding claim, wherein the rows of perforations are distributed circumferentially along the circumferential wall.

5. The apparatus according to any preceding claim, wherein, for each of the rows, at least one of the perforations in the row is larger than other perforations in the row.

6. The apparatus according to claim 5, wherein at least a proximalmost one of the perforations in the row is larger than the other perforations in the row.

7. The apparatus according to any preceding claim, wherein the inner electrode comprises a wire.

8. The apparatus according to any preceding claim, wherein the inner electrode comprises an electrically-conducting coil.

9. The apparatus according to any preceding claim, wherein the inner electrode comprises an electrically-conducting mesh.

10. The apparatus according to any preceding claim, wherein the inner electrode comprises an electrically-conducting tube.

11. The apparatus according to claim 10, wherein the perforations are first perforations, and wherein the electrically-conducting tube is shaped to define one or more second perforations that are aligned with the first perforations.

12. The apparatus according to any preceding claim, further comprising a pump, configured to facilitate the removal of the thrombus by applying suction to the thrombus, via the perforations, while the voltage is applied.

## Patentansprüche

1. Vorrichtung (20) zum Entfernen eines Thrombus (66) aus einem Körper eines Patienten, die Vorrichtung umfassend:
ein elektrisch isolierendes Rohr (22), das ein distales Ende (30), aufweisend eine Umfangswand (38) umfasst, die so geformt ist, dass sie mehrere Perforationen (34) definiert, die in einer oder mehreren Reihen (58) angeordnet sind, und das zum Einführen in den Körper des Patienten konfiguriert ist;
eine äußere Elektrode (24), die über dem distalen Ende des elektrisch isolierenden Rohrs eingerichtet ist, ohne die Perforationen vollständig zu bedecken, so dass ein proximales Elektrodenende (52) der äußeren Elektrode proximal zu einer proximalsten der Perforationen und ein distales Elektrodenende (54) der äußeren Elektrode distal zu einer distalsten der Perforationen liegt, und die so konfiguriert ist, dass sie zumindest teilweise innerhalb des Thrombus liegt, während das elektrisch isolierende Rohr innerhalb des Körpers des Patienten ist; und eine innere Elektrode (28), die so konfiguriert ist, dass sie innerhalb des Rohrs gegenüber den Perforationen liegt, während die äußere Elektrode zumindest teilweise innerhalb des Thrombus liegt;
wobei die äußere Elektrode so konfiguriert ist, dass sie den Thrombus anzieht, während die äußere Elektrode zumindest teilweise innerhalb des Thrombus liegt und die innere Elektrode gegenüber den Perforationen liegt, wenn eine positive Spannung zwischen der äußeren Elektrode und der inneren Elektrode angelegt wird, so dass elektrischer Strom durch die Perforationen fließt;
**dadurch gekennzeichnet, dass** die äußere Elektrode eine Spule umfasst, die um das distale Ende des elektrisch isolierenden Rohrs gewickelt ist; und
dass die Spule um das distale Ende des elektrisch isolierenden Rohrs zwischen den Perforationen gewickelt ist.

2. Vorrichtung nach Anspruch 1, wobei das distale Ende des elektrisch isolierenden Rohrs so geformt ist, dass es eine distale Öffnung definiert.

3. Vorrichtung nach Anspruch 1, wobei das distale Ende des elektrisch isolierenden Rohrs verschlossen ist.

4. Vorrichtung nach einem der vorhergehenden Ansprüche, wobei die Reihen von Perforationen in Umfangsrichtung entlang der Umfangswand verteilt sind.

5. Vorrichtung nach einem der vorhergehenden Ansprüche, wobei für jede der Reihen mindestens eine der Perforationen in der Reihe größer ist als andere Perforationen in der Reihe.

6. Vorrichtung nach Anspruch 5, wobei mindestens eine der proximalsten Perforationen in der Reihe größer ist als die anderen Perforationen in der Reihe.

7. Vorrichtung nach einem der vorhergehenden Ansprüche, wobei die innere Elektrode einen Draht umfasst.

8. Vorrichtung nach einem der vorhergehenden Ansprüche, wobei die innere Elektrode eine elektrisch leitende Spule umfasst.

9. Vorrichtung nach einem der vorhergehenden Ansprüche, wobei die innere Elektrode ein elektrisch leitendes Netz umfasst.

10. Vorrichtung nach einem der vorhergehenden Ansprüche, wobei die innere Elektrode ein elektrisch leitendes Rohr umfasst.

11. Vorrichtung nach Anspruch 10, wobei die Perforationen erste Perforationen sind, und wobei das elektrisch leitende Rohr so geformt ist, dass es eine oder mehrere zweite Perforationen definiert, die mit den ersten Perforationen ausgerichtet sind.

12. Vorrichtung nach einem der vorhergehenden Ansprüche, ferner umfassend eine Pumpe, die so konfiguriert ist, dass sie die Entfernung des Thrombus erleichtert, indem sie über die Perforationen einen Sog auf den Thrombus ausübt, während die Spannung angelegt wird.

## Revendications

1. Appareil (20) destiné à l'élimination d'un thrombus (66) à partir d'un corps d'un sujet, l'appareil comprenant :
un tube électriquement isolant (22), qui comprend une extrémité distale (30) présentant une paroi circonférentielle (38) qui est façonnée pour définir de multiples perforations (34), disposées en une ou plusieurs rangées (58), configuré pour insertion dans le corps du sujet ;
une électrode externe (24), disposée sur l'extrémité distale du tube électriquement isolant sans couvrir entièrement les perforations, de sorte qu'une extrémité proximale (52) d'électrode de l'électrode externe est proximale par rapport à une la plus proximale des perforations et une extrémité distale (54) d'électrode de l'électrode externe est distale par rapport à une la plus distale des perforations, et configurée pour se trouver au moins partiellement à l'intérieur du thrombus alors que le tube électriquement isolant est à l'intérieur du corps du sujet ; et
une électrode interne (28), configurée pour se trouver, à l'intérieur du tube, à l'opposé des perforations, alors que l'électrode externe se trouve au moins partiellement à l'intérieur du thrombus ;
l'électrode externe étant configurée pour attirer le thrombus alors que l'électrode externe se trouve au moins partiellement à l'intérieur du thrombus et l'électrode interne se trouve à l'opposé des perforations, lorsqu'une tension positive est appliquée entre l'électrode externe et l'électrode interne de sorte que du courant électrique circule à travers les perforations ;
**caractérisé en ce que** l'électrode externe comprend une spirale qui est enroulée autour de l'extrémité distale du tube électriquement isolant ; et
**en ce que** la spirale est enroulée autour de l'extrémité distale du tube électriquement isolant, entre les perforations.

2. Appareil selon la revendication 1, dans lequel l'extrémité distale du tube électriquement isolant est façonnée pour définir un orifice distal.

3. Appareil selon la revendication 1, dans lequel l'extrémité distale du tube électriquement isolant est fermée.

4. Appareil selon l'une quelconque des revendications précédentes, dans lequel les rangées de perforations sont réparties circonférentiellement le long de la paroi circonférentielle.

5. Appareil selon l'une quelconque des revendications précédentes, dans lequel, pour chacune des rangées, au moins une des perforations dans la rangée est plus grande que d'autres perforations dans la rangée.

6. Appareil selon la revendication 5, dans lequel au moins une la plus proximale des perforations dans la rangée est plus grande que les autres perforations dans la rangée.

7. Appareil selon l'une quelconque des revendications précédentes, dans lequel l'électrode interne comprend un fil métallique.

8. Appareil selon l'une quelconque des revendications précédentes, dans lequel l'électrode interne comprend un enroulement électriquement conducteur.

9. Appareil selon l'une quelconque des revendications précédentes, dans lequel l'électrode interne comprend un treillis électriquement conducteur.

10. Appareil selon l'une quelconque des revendications précédentes, dans lequel l'électrode interne comprend un tube électriquement conducteur.

11. Appareil selon la revendication 10, dans lequel les perforations sont des premières perforations, et dans lequel le tube électriquement conducteur est façonné pour définir une ou plusieurs secondes perforations qui sont alignées avec les premières perforations.

12. Appareil selon l'une quelconque des revendications précédentes, comprenant en outre une pompe, configurée pour faciliter l'élimination du thrombus en appliquant une aspiration sur le thrombus, via les perforations, pendant que la tension est appliquée.
